# FASCICULE DE BREVET EUROPEEN

(11) **EP 3 119 478 B1**
(45) Date de publication et mention de la délivrance du brevet: **04.07.2018**
(21) Numéro de dépôt: 15713986.6
(22) Date de dépôt: 05.03.2015
(51) Int. Cl.: A61K 8/02, A61K 8/11, A61Q 19/00

(54) **DISPOSITIF CONSOMMABLE ANHYDRE DESTINE A LA PREPARATION EXTEMPORANEE D'UNE COMPOSITION COSMETIQUE MONODOSE**
ANYHDRISCHE VERBRAUCHBARE VORRICHTUNG FÜR DIE IMPROVISIERTE ZUBEREITUNG EINER KOSMETISCHEN EINZELDOSISZUSAMMENSETZUNG
ANHYDROUS CONSUMABLE DEVICE FOR THE EXTEMPORANEOUS PREPARATION OF A SINGLE-DOSE COSMETIC COMPOSITION

(30) Priorité: 21.03.2014 FR 1452377
(43) Date de publication de la demande: 25.01.2017
(73) Titulaire: GATTEFOSSE HOLDING, 69800 St Priest (FR)
(72) Inventeur: DEMARNE, Frédéric, F-13008 Marseille (FR)
(74) Mandataire: Cabinet Laurent & Charras
(86) Numéro de dépôt international: PCT/FR2015/050549
(87) Numéro de publication internationale: WO 2015/140444

(56) Documents cités:
- EP-A1- 0 453 683
- CH-A5- 663 725
- GB-A- 2 118 961
- US-A- 4 292 304
- US-A- 5 326 564
- US-A1- 2004 234 590

## Description

La présente invention concerne un dispositif consommable anhydre comprenant une phase grasse destiné à la préparation extemporanée uniquement par ajout d'eau, d'une émulsion cosmétique monodose. Elle a également pour objet un procédé de préparation extemporanée d'une émulsion cosmétique monodose mettant en oeuvre le dispositif de l'invention. Ce procédé est destiné à être mis en oeuvre directement par le consommateur du produit cosmétique, juste avant utilisation.

Les produits cosmétiques classiques sont fabriqués industriellement en usine, conditionnés dans des contenants de taille variable, puis envoyés vers des points de vente où ils sont vendus au consommateur final après un temps de stockage plus ou moins long. Les contenants utilisés pour les produits cosmétiques (tubes, pots, flacons ou autres) présentent généralement une capacité qui correspond à plusieurs semaines d'utilisation du produit cosmétique. Après achat, le consommateur conserve donc le produit cosmétique chez lui pendant une durée souvent supérieure à plusieurs mois. Ce stockage se fait à température ambiante, sans aucune précaution particulière, le produit cosmétique pouvant ainsi être exposé des températures importantes en été ou s'il est conservé à proximité d'un radiateur.

Or, les produits cosmétiques, qui sont le plus souvent des émulsions de type huile dans l'eau, ont une durée de vie limitée. En effet, les deux phases de ces émulsions ont tendance à se séparer au bout d'un certain temps. Ce phénomène de vieillissement est accéléré lorsque le produit est exposé à la chaleur. En outre, les principes actifs utilisés pour la fabrication de ces cosmétiques sont souvent instables et se décomposent lorsqu'ils sont en contact prolongé avec la phase aqueuse de l'émulsion.

Pour prolonger la durée de vie des produits cosmétiques en conservant la qualité de l'émulsion et son efficacité, les produits cosmétiques contiennent habituellement des conservateurs dans le but de les stabiliser mais également d'éviter le développement de microorganismes. Ces conservateurs, qui sont souvent des produits chimiques de synthèse tels que des parabens ou autre, sont assez mal vus par les consommateurs actuels. Ils sont considérés comme toxiques, responsables d'allergie et soupçonnés d'effets néfastes sur la santé.

Selon la tendance actuelle, un grand nombre de consommateurs recherchent des produits cosmétiques à base d'ingrédients naturels, avec le moins possible d'excipients chimiques et de conservateurs. Cependant, la suppression des conservateurs est incompatible avec la façon actuelle dont les produits cosmétiques sont fabriqués, distribués, stockés et utilisés par les consommateurs.

Pour satisfaire ces consommateurs, on a proposé des produits cosmétiques à fabriquer soi-même, chez-soi, en suivant une recette, à partir d'ingrédients achetés séparément. Cependant, ces préparations sont assez complexes à réaliser. Elles nécessitent du temps et du matériel parfois onéreux. En outre, il faut acheter, puis stocker, un grand nombre de matières premières en quantité relativement importante. Du fait de l'investissement financier nécessaire et de la complexité des préparations proposées qui découragent souvent les consommateurs initialement intéressés, cette tendance est restée marginale pour l'instant. En outre, elle ne résout pas le problème de la durée de vie limitée des produits cosmétiques fabriqués lorsqu'ils ne contiennent pas de conservateurs.

Pour faciliter la fabrication des produits cosmétiques par les consommateurs, on a proposé dans l'art antérieur des procédés de fabrication simplifiés.

On connait ainsi par exemple le brevet EP 2.038.189 B1 qui décrit un procédé pour la production d'un produit cosmétique selon lequel le consommateur fabrique lui-même ce produit cosmétique en quantité correspondant aux habitudes de son foyer. Il utilise pour cela une machine dans laquelle il place une ou plusieurs unités de conditionnement renfermant tous les ingrédients cosmétiques nécessaires à la fabrication du produit en quantités préalablement mesurées. La machine réalise ensuite automatiquement le mélange de tous les ingrédients cosmétiques contenus dans les unités de conditionnement, avec de l'eau, sans que le consommateur n'ait besoin de mesurer les quantités ou de peser les ingrédients. Ce procédé simplifie la fabrication d'un produit cosmétique par le consommateur car il n'a plus besoin de peser ou de mesurer les quantités des ingrédients qui lui sont fournis. C'est la machine qui réalise automatiquement le mélange de ces ingrédients avec de l'eau. Cependant, ce procédé nécessite l'achat par le consommateur d'une machine spécifique, indispensable pour réaliser le procédé divulgué, dont le coût est important. De plus, cette machine est encombrante, consomme de l'énergie pour son fonctionnement et requiert un entretien. En outre, les unités de conditionnement prévues, qui comportent plusieurs compartiments étanches distincts afin de séparer les différents composants, sont complexes à réaliser ce qui se traduit par coût de revient important. Pour le consommateur, le prix d'achat des ingrédients dans ces unités de conditionnement est donc élevé et comparativement beaucoup plus cher que le coût des produits cosmétiques classiques disponibles dans le commerce.

Par ailleurs, les consommateurs intéressés par la fabrication à domicile de leurs produits cosmétiques sont pour la plupart des personnes qui recherchent pour leurs cosmétiques des produits naturels et veulent contrôler et limiter l'utilisation des ingrédients chimiques et des conservateurs. Si leur motivation principale est la préservation de leur santé, elle va généralement de pair avec une préoccupation écologique de préservation de l'environnement. Or, après utilisation, les unités de conditionnement vides constituent des déchets nombreux, encombrants et généralement non recyclables qui posent un problème environnemental et heurtent la sensibilité écologique de ces consommateurs potentiels qui sont généralement attachés à la lutte contre le suremballage.

Enfin, ce brevet antérieur prévoit de préparer les produits cosmétiques en quantité domestique, c'est-à-dire en quantité comparable à celle des conditionnements dans lesquels le produit est habituellement vendu au consommateur final dans les commerces classiques. Une fois préparé, le consommateur aura donc à le conserver pendant plusieurs semaines, ce qui peut se révéler problématique en l'absence de conservateurs.

On connait également le procédé et le kit divulgués dans la demande de brevet US 2004/0202684. Selon ce procédé, pour obtenir un produit cosmétique, le consommateur mélange lui-même dans un récipient fourni dans le kit, le contenu de deux ou trois contenants également fournis dans le kit. Le premier de ces contenants contient un mélange de principes actifs cosmétiques anhydres, le deuxième un liquide aqueux et le troisième un agent épaississant et/ou gélifiant que le consommateur peut ajouter en quantité variable afin d'obtenir un produit cosmétique avec la consistance finale qu'il désire. Selon les variantes, l'agent épaississant peut également être mélangé aux principes actifs anhydres du premier contenant. Dans ce cas, le kit ne comprend plus que deux contenants.

Le but de ce procédé est de pouvoir personnaliser le produit cosmétique fabriqué en y ajoutant d'autres ingrédients issus des boissons ou de l'alimentation (jus de fruits ou de légumes frais, thé, café, lait, vin...) dont les composants sont particulièrement instables.

Ce procédé peut avantageusement être réalisé manuellement sans utilisation d'une machine spécifique, ce qui le rend plus abordable financièrement pour les consommateurs. Cependant, la méthode est plus compliquée pour l'utilisateur qui doit rajouter des ingrédients non fournis dans le kit, pour lequel il doit déterminer et mesurer lui-même les quantités à rajouter. De même, les ingrédients fournis dans les contenants du kit ne doivent pas forcément être utilisés en totalité et doivent donc être mesurés avant ajout, puis conservés.

En outre, le problème des déchets générés par les contenants vides après utilisation des ingrédients, n'est pas résolu. La quantité d'emballage à jeter est encore augmentée par le fait que, selon ce procédé, même l'eau nécessaire à la préparation du produit cosmétique est conditionnée dans un contenant fourni dans le kit dont il faut se débarrasser après usage.

Le document CH 663725 A5 décrit un procédé de fabrication instantanée de pommade consistant à mélanger des sachets-doses distincts contenant des ingrédients constitutifs d'une phase grasse ou d'une phase aqueuse. Les sachets sont déversés dans un récipient du type tube dans lequel le mélange est effectué. L'opérateur doit donc sélectionner les différents sachets et mélanger leur contenu pour obtenir la composition finale à partir d'une recette prédéterminée.

Le document GB 2 118 961 A décrit une préparation pour le bain se présentant sous la forme de sachets contenant essentiellement des tensio-actifs. Ces sachets sont fabriqués en PVA et sont destinés à se dissoudre dans l'eau du bain.

Le document US 5326564 décrit des capsules double-compartiments, dont un des compartiments peut être rempli d'une composition anhydre. L'enveloppe est réutilisable.

Le document US 4 292 304 A décrit un dentifrice anhydre à base d'huile, stockée dans des capsules de gélatine destinées à être ingérées. La capsule sert uniquement à l'emballage du dentifrice.

Le document EP 453 683 A1 décrit une composition cosmétique se présentant sous la forme d'une capsule à base de gélatine contenant une composition cosmétique. La capsule est uniquement utilisée comme contenant.

Le document US 2004/0234590 A1 décrit des capsules destinées à être ingérées. L'enveloppe est destinée à se dissoudre au contact d'une phase aqueuse.

Le but de l'invention est de proposer un procédé alternatif de préparation d'un produit cosmétique réalisable par l'utilisateur, qui ne présente pas les inconvénients des procédés antérieurs précités.

Le procédé selon l'invention est extrêmement facile à mettre en oeuvre, par une simple opération de mélange et sans qu'il soit nécessaire d'utiliser une machine. Il ne nécessite aucune opération compliquée de préparation des ingrédients ou de mesure de leurs quantités respectives par l'utilisateur.

Ce procédé, rapide et économique, est en outre écologique car il ne génère aucun déchet.

Le procédé selon l'invention permet à l'utilisateur de préparer, de façon extemporanée, uniquement la dose de produit cosmétique nécessaire, juste avant son application. Le produit cosmétique ainsi fabriqué n'a pas vocation à être conservé et est prévu pour être utilisé tout de suite après sa fabrication.

Son efficacité est donc maximale et l'utilisation de conservateurs, voire de stabilisants n'est absolument pas nécessaire. Il est ainsi possible de fabriquer très facilement des produits cosmétiques sans conservateurs, parfaitement adaptés aux attentes des consommateurs qui recherchent des produits cosmétiques à base de principes actifs « purs », c'est-à-dire sans additifs superflus.

Plus précisément, l'invention propose un dispositif consommable anhydre comprenant une phase grasse, le dispositif étant destiné à la préparation extemporanée, uniquement par ajout d'eau, d'une émulsion cosmétique monodose.

En d'autres termes, l'invention consiste en un dispositif destiné à la préparation, juste avant application sur la peau, les phanères ou les muqueuses, d'une composition cosmétique monodose à usage unique, c'est-à-dire d'une composition cosmétique applicable immédiatement et dans sa totalité sur la peau, les phanères ou les muqueuses.

L'émulsion cosmétique est obtenue à l'issue du mélange du dispositif de l'invention avec de l'eau. Aucun ingrédient supplémentaire n'est nécessaire.

Le terme « monodose » s'entend d'une quantité d'émulsion cosmétique suffisante pour être appliquée dans sa totalité et en une seule fois sur la peau, les phanères ou les muqueuses.

De même, le terme « consommable », signifie que l'ensemble des éléments constitutifs du dispositif participent à la formation de l'émulsion cosmétique finale.

Le terme « anhydre » désigne une composition exempte ou pratiquement exempte d'eau.

Le dispositif consommable anhydre se présente en pratique sous la forme d'une enveloppe, fermée, constituant un ingrédient de l'émulsion cosmétique et renfermant une phase grasse correspondant au reste des ingrédients de ladite émulsion cosmétique, à l'exception de l'eau.

L'invention concerne dons un dispositif consommable anhydre se présentant sous la forme d'une enveloppe fermée renfermant une phase grasse, l'enveloppe et la phase grasse constituant les ingrédients nécessaires et dans une quantité adaptée à la préparation extemporanée, uniquement par ajout d'eau, d'une émulsion cosmétique à usage unique.

Selon une caractéristique essentielle de l'invention, tous les constituants du dispositif consommable anhydre participent à la formule cosmétique finale, y compris l'enveloppe.

Selon l'invention, lorsque le dispositif est mélangé avec de l'eau en quantité adaptée, il forme une émulsion de type huile dans l'eau dans laquelle l'enveloppe est entièrement consommée.

En fonction des modes de réalisation de l'invention, l'enveloppe peut être une gélule, une capsule ou un sachet scellé.

Les ingrédients contenus dans la phase grasse sont en pratique choisis parmi : les principes actifs cosmétiques, les huiles synthétiques, les huiles végétales, les émollients, les filtres anti-UV, les agents texturants, les agents épaississants, les agents gélifiants, les pigments ou colorants, les arômes ou parfums, les tensioactifs, sans que cette liste ne soit limitative. L'homme du métier saura sélectionner les ingrédients nécessaires à la formation d'une émulsion après contact avec l'eau.

Selon une caractéristique essentielle, l'ensemble du dispositif consommable, enveloppe et contenu, sont nécessaires à l'obtention du produit cosmétique fini, et contribuent tous les deux aux qualités techniques et aux propriétés sensorielles du produit cosmétique fini. L'enveloppe sert entres autres d'agent gélifiant et/ou d'agent de texture ; elle est entièrement consommée et permet que le procédé selon l'invention ne génère pas de déchet.

Pour ce faire, l'enveloppe peut être réalisée à partir d'un ou plusieurs composants parmi la gélatine, l'hydroxy-propyl-méthyl-cellulose, les polymères naturels ou synthétiques, le pullulane ou la gomme gellane. On peut notamment utiliser à cet effet les capsules commercialisées par la société CAPSUGEL-France sous les noms commerciaux de « Plantacaps™ » en pullulane, « Vcaps® » en hydroxy-propyl-méthyl-cellulose, « Licaps® » en gélatine ou « DRcaps™ » en un mélange de gomme gellane (5%) et d'hydroxy-propyl-méthyl-cellulose (qsp 100%).

L'invention concerne également un kit pour la mise en oeuvre du procédé de préparation selon l'invention, qui se caractérise en ce qu'il comprend :
- au moins un dispositif consommable anhydre; et
- un récipient adapté pour réaliser la mise en contact entre l'eau, le dispositif, et pour réaliser le mélange permettant de former l'émulsion.

Dans mode de réalisation particulier, le récipient contenu dans ce kit peut présenter au moins un repère visuel ou une graduation indiquant à l'utilisateur la quantité d'eau à verser dans le récipient.

Selon un autre mode de réalisation, ce récipient peut comprendre un moyen d'obturation qui, lorsqu'il est retiré ou se trouve en position ouverte permet l'introduction de l'eau et du dispositif consommable ahnydre, et qui lorsqu'il est mis en place ou se trouve en position fermée rend le récipient étanche et permet de réaliser le mélange en secouant le récipient.

Selon encore un autre mode de réalisation, ce récipient peut comprendre au moins un moyen d'amélioration du mélange qui facilite la formation de l'émulsion.

L'invention a également pour objet un procédé de préparation extemporanée d'une émulsion cosmétique monodose à usage unique mettant en oeuvre le dispositif de l'invention. Ce procédé est destiné à être mis en oeuvre par le consommateur de l'émulsion cosmétique juste avant l'application de ladite émulsion sur la peau, les phanères ou les muqueuses.

Le procédé est caractérisé en ce qu'il comprend les étapes suivantes :
- fournir un dispositif consommable anhydre comprenant une phase grasse,
- fournir de l'eau, en quantité prédéterminée, correspondant à la quantité nécessaire à la préparation de l'émulsion cosmétique à usage unique;
- mettre l'eau en contact avec le dispositif et mélanger l'ensemble pour former une émulsion cosmétique à usage unique monodose applicable directement, immédiatement et dans sa totalité sur la peau, les phanères ou les muqueuses.

Le procédé selon l'invention permet de réaliser simplement tout produit cosmétique se présentant sous la forme d'une émulsion de type « huile dans l'eau », qu'il s'agisse d'une crème, d'un lait, d'un gel, ou de toute autre forme d'émulsion envisageable pour la réalisation d'un produit cosmétique destiné à être appliqué sur une partie quelconque du corps humain, notamment la peau (du corps ou du visage), les cheveux ou les ongles ou les muqueuses.

Selon d'autres modes de réalisation de l'invention, on peut chauffer l'eau avant de la mettre en contact avec le dispositif, et/ou chauffer le mélange pendant l'étape de mélange, pour faciliter la formation de l'émulsion, et/ou refroidir ou laisser refroidir l'émulsion obtenue avant de l'appliquer sur la peau. La température de l'eau peut être de l'ordre de 60 à 70°C.

Le procédé selon l'invention permet ainsi par exemple de réaliser un produit hydratant (crème pour le visage de jour ou de nuit, lait corporel, crème pour les mains, les lèvres...), produit traitant (antirides, anti-acné, anticernes...), un produit capillaire (antipelliculaire, antichute, fortifiant, colorant ou décolorant, démêlant...), un produit de protection solaire ou auto-bronzant, un gommage pour le corps ou le visage, un masque pour la peau ou les cheveux, un produit de maquillage ou de démaquillage, un produit pour le rasage ou l'épilation, un produit parfumant, un produit de toilette tels qu'un shampoing, un après-shampoing, un gel douche, un bain moussant ou autres, un gel de massage, ou tout autre produit cosmétique.

Le procédé selon l'invention est destiné à être mis en oeuvre directement par l'utilisateur du produit cosmétique, à son domicile. Il est de ce fait particulièrement simple, de manière à pouvoir être réalisé par n'importe qui, sans formation particulière, ni habitude ou entraînement, et sans aucun matériel spécifique.

Il pourra néanmoins, sans sortir du cadre de la présente invention, être réalisé par un professionnel dans un institut de beauté, un salon de coiffure, de manucure, de maquillage ou d'esthétique, un centre de bien-être, de relaxation ou de massage, un magasin de produits cosmétiques ou toute autre structure similaire dans laquelle un produit cosmétique pourrait être préparé en vue d'être appliqué sur le corps d'un consommateur.

Les ingrédients contenus dans la phase grasse du dispositif anhydre peuvent se présenter sous une forme solide ou liquide ou pâteux. Dans tous les cas, le dispositif anhydre est exempte ou pratiquement exempte d'eau, l'eau nécessaire à la préparation de l'émulsion étant rajoutée par la suite.

A partir du dispositif consommable anhydre, le procédé selon l'invention est très facile à mettre en oeuvre. Il suffit ainsi de fournir une quantité d'eau adaptée à la préparation d'une dose du produit cosmétique souhaité. Cette quantité d'eau est prédéterminée à l'avance et dépend de la nature et de la masse de la composition anhydre, incluant l'enveloppe. Elle dépend en outre de la nature du produit cosmétique que l'on souhaite obtenir, l'obtention par exemple d'une dose de lait nécessitant évidemment plus d'eau que l'obtention d'une dose de crème.

Au contact de l'eau, l'enveloppe de la composition anhydre gonfle, se désagrège et/ou se dissout. Les autres ingrédients sont alors libérés et se retrouve dans l'eau. En mélangeant le dispositif anhydre dans l'eau, on forme une émulsion de type huile dans l'eau qui constitue le produit cosmétique final. La monodose de produit cosmétique obtenue est alors prête à être utilisée.

L'invention et les avantages qui en découlent ressortiront bien des exemples de réalisation ci-après.

5 mélanges d'ingrédients cosmétiques distincts ont été préparés. Chaque mélange a été introduit dans 4 capsules différentes.

Les capsules utilisées sont les suivantes et sont fournies par la société CAPSUGEL : Plantcaps® (100% pullulan)
Vcaps® (100% hypromellose)
Licaps® (gélatin)
DRcaps® (5% gomme de gellane + hypromellose qsp 100%)

### Exemple 1 :

On prépare quatre formules identiques contenant la composition détaillée dans le tableau ci-dessous.

| **Ingrédients** | **Fonction** | **Composition** (en pourcentage massique) |
|---|---|---|
| Emulium Delta | émulsifiant | 37,5 |
| Labrafac CC | huile | 37,5 |
| DPPG | agent texturant | 25 |

Les compositions ainsi obtenues sont introduites dans chacune des 4 capsules précitées. Chaque composition anhydre ainsi obtenue est mélangée à 6 g d'eau chaude à 65°C.

Avec la capsule Licaps®, on obtient une monodose de crème.
Avec la capsule Vcaps®, on obtient une monodose de lait.
Avec la capsule Plantcaps®, on obtient une monodose de crème.
Avec la capsule DRcaps®, on obtient une monodose de crème.

### Exemple 2 :

On prépare quatre formules identiques contenant la composition détaillée dans le tableau ci-dessous.

| **Ingrédients** | **Fonction** | **Composition** (en pourcentage massique) |
|---|---|---|
| Emulium Delta | émulsifiant | 23 |
| Labrafac CC | huile | 22 |
| Huile de jojoba | huile | 11 |
| DPPG | agent texturant | 15 |
| Xiameter PMX-0345 | agent texturant | 27 |
| Gatuline intense | principe actif | 2 |

Les compositions ainsi obtenues sont introduites dans chacune des 4 capsules précitées. Chaque composition anhydre ainsi obtenue est mélangée à 5 g d'eau chaude à 65°C.

Avec la capsule Licaps®, on obtient une monodose de crème.
Avec la capsule Vcaps®, on obtient une monodose de lait.
Avec la capsule Plantcaps®, on obtient une monodose de crème.
Avec la capsule DRcaps®, on obtient une monodose de crème.

### Exemple 3 :

On prépare quatre formules identiques contenant la composition détaillée dans le tableau ci-dessous.

| **Ingrédients** | **Fonction** | **Composition** (en pourcentage massique) |
|---|---|---|
| Emulium 22 | émulsifiant | 25 |
| MOD | émollient | 29 |
| Cetiol OE | émollient | 17 |
| Geleol | agent texturant | 12 |
| Dimethicone Fluid 100 | agent texturant | 17 |

Les compositions ainsi obtenues sont introduites dans chacune des 4 capsules précitées. Chaque composition anhydre ainsi obtenue est mélangée à 5 g d'eau chaude à 65°C.

Avec la capsule Licaps®, on obtient une monodose de crème.
Avec la capsule Vcaps®, on obtient une monodose de lait.
Avec la capsule Plantcaps®, on obtient une monodose de crème.
Avec la capsule DRcaps®, on obtient une monodose de crème.

### Exemple 4 :

On prépare quatre formules identiques contenant la composition détaillée dans le tableau ci-dessous.

| **Ingrédients** | **Fonction** | **Composition** (en pourcentage massique) |
|---|---|---|
| Emulium kappa | émulsifiant | 18 |
| Cetiol OE | émollient | 23 |
| Eutanol G | émollient | 27 |
| Isostéaryl isostéarate | émollient | 18 |
| Lipocire A | agent texturant | 14 |

Les compositions ainsi obtenues sont introduites dans chacune des 4 capsules précitées. Chaque composition anhydre ainsi obtenue est mélangée à 5 g d'eau chaude à 65°C.

Avec la capsule Licaps®, on obtient une monodose de crème.
Avec la capsule Vcaps®, on obtient une monodose de lait.
Avec la capsule Plantcaps®, on obtient une monodose de crème.
Avec la capsule DRcaps®, on obtient une monodose de lait crème.

### Exemple 5 :

On prépare quatre formules identiques contenant la composition détaillée dans le tableau ci-dessous.

| **Ingrédients** | **Fonction** | **Composition** (en pourcentage massique) |
|---|---|---|
| Emulium kappa | émulsifiant | 22 |
| Huile de jojoba | huile | 27 |
| Cetiol OE | émollient | 30 |
| Geleol | agent texturant | 9 |
| Lipocire A | agent texturant | 6 |
| Huile essentielle de lavande | principe actif | 6 |

Les compositions ainsi obtenues sont introduites dans chacune des 4 capsules précitées. Chaque composition anhydre ainsi obtenue est mélangée à 5 g d'eau chaude à 65°C.

Avec la capsule Licaps®, on obtient une monodose de crème.
Avec la capsule Vcaps®, on obtient une monodose de lait.
Avec la capsule Plantcaps®, on obtient une monodose de crème.
Avec la capsule DRcaps®, on obtient une monodose de crème.

De manière évidente, l'invention ne se limite pas aux modes de réalisation préférentiels décrits précédemment, l'homme du métier pouvant y apporter de nombreuses modifications et imaginer d'autres variantes sans sortir ni de la portée, ni du cadre de l'invention définis par les revendications.

## Revendications

1. Dispositif anhydre se présentant sous la forme d'une enveloppe fermée renfermant une phase grasse, **caractérisé en ce que** :
- l'enveloppe et la phase grasse constituent les ingrédients nécessaires et dans une quantité adaptée à la préparation extemporanée, uniquement par ajout d'eau, d'une émulsion cosmétique à usage unique,
- l'enveloppe sert d'agent gélifiant et/ou d'agent de texture, et
- l'émulsion est sous forme d'une crème, d'un lait ou d'un gel.

2. Dispositif selon la revendication 1, **caractérisé en ce qu'**il s'agit d'une gélule, d'une capsule, ou d'un sachet scellé.

3. Dispositif selon l'une des revendications précédentes, **caractérisé en ce que** les ingrédients contenus dans la phase grasse sont choisis parmi les principes actifs cosmétiques, les huiles synthétiques, les huiles végétales, les émollients, les filtres anti-UV, les agents texturants, les agents épaississants, les agents gélifiants, les pigments ou colorants, les arômes ou parfums, les tensioactifs.

4. Dispositif selon l'une des revendications 1 à 3, **caractérisé en ce que** l'enveloppe est réalisée à partir d'un ou plusieurs composants parmi la gélatine, l'hydroxy-propyl-méthyl-cellulose, les polymères naturels ou synthétiques, le pullulane ou la gomme gellane.

5. Dispositif selon l'une des revendications précédentes, **caractérisé en ce que** les ingrédients contenus dans la phase grasse se présentent sous une forme solide ou liquide ou pâteux.

6. Procédé de préparation extemporanée d'une émulsion cosmétique à usage unique mettant en oeuvre le dispositif objet de l'une des revendications 1 à 5.

7. Procédé selon la revendication 6, **caractérisé en ce qu'**il comprend les étapes suivantes :
• fournir le dispositif consommable anhydre comprenant une phase grasse,
• fournir de l'eau, en quantité prédéterminée, correspondant à la quantité nécessaire à la préparation de l'émulsion cosmétique à usage unique;
• mettre l'eau en contact avec le dispositif et mélanger l'ensemble pour former une émulsion cosmétique usage unique applicable directement, immédiatement et dans sa totalité sur la peau, les phanères ou les muqueuses.

8. Procédé selon la revendication 8 **caractérisé en ce que** l'on chauffe l'eau avant de la mettre en contact avec le dispositif consommable anhydre et/ou chauffer le mélange pendant l'étape de mélange, pour faciliter la formation de l'émulsion, et/ou refroidir ou laisser refroidir l'émulsion obtenue avant de l'appliquer sur la peau.

9. Procédé selon l'une des revendications 7 à 8, **caractérisé en ce que** l'émulsion cosmétique obtenue est une crème ou un lait ou un gel.

## Patentansprüche

1. Wasserfreie Vorrichtung, in Form einer geschlossenen Hülle vorliegend, die eine Fettphase enthält, **dadurch gekennzeichnet, dass**:
- die Hülle und die Fettphase die erforderlichen Bestandteile bilden, und zwar in einer Menge, die für die magistrale Zubereitung einer kosmetischen Emulsion zum einmaligen Gebrauch allein durch Hinzufügen von Wasser angepasst ist,
- die Hülle als Gelier- und/oder Texturmittel dient, und
- die Emulsion in Form einer Creme, einer Milch oder eines Gels vorliegt.

2. Vorrichtung gemäß Anspruch 1, **dadurch gekennzeichnet, dass** es sich um eine Gelatinekapsel, eine Kapsel oder einen versiegelten Beutel handelt.

3. Vorrichtung gemäß einem der vorhergehenden Ansprüche, **dadurch gekennzeichnet, dass** die in der Fettphase enthaltenen Inhaltsstoffe ausgewählt sind aus kosmetischen Wirkstoffen, synthetischen Ölen, Pflanzenölen, Weichmachern, Anti-UV-Filtern, Texturierungsmitteln, Verdickungsmitteln, Geliermitteln, Pigmenten oder Farbstoffen, Aromen oder Duftstoffen, Tensiden.

4. Vorrichtung gemäß einem der Ansprüche 1 bis 3, **dadurch gekennzeichnet, dass** die Hülle aus einem oder mehreren Bestandteilen aus Gelatine, Hydroxypropylmethylcellulose, natürlichen oder synthetischen Polymeren, Pullulan oder Gellan-Gummi hergestellt ist.

5. Vorrichtung gemäß einem der vorhergehenden Ansprüche, **dadurch gekennzeichnet, dass** die in der Fettphase enthaltenen Inhaltsstoffe in fester oder flüssiger oder pastöser Form vorliegen.

6. Verfahren zur magistralen Zubereitung einer kosmetischen Emulsion zum einmaligen Gebrauch, welches die Vorrichtung gemäß einem der Ansprüche 1 bis 5 umsetzt.

7. Verfahren gemäß Anspruch 6, **dadurch gekennzeichnet, dass** es die folgenden Schritte umfasst:
. das Bereitstellen der wasserfreien verbrauchbaren Vorrichtung, umfassend eine Fettphase,
. das Bereitstellen des Wassers in einer vorbestimmten Menge, die der Menge entspricht, welche zur Herstellung der kosmetischen Emulsion zum einmaligen Gebrauch erforderlich ist;
. das Inkontaktbringen des Wassers mit der Vorrichtung und das Mischen des Ganzen, um eine kosmetische Emulsion zum einmaligen Gebrauch zu bilden, die direkt, sofort und zur Gänze auf die Haut, die Hautanhangsgebilde oder die Schleimhäute anwendbar ist.

8. Verfahren gemäß Anspruch 8, **dadurch gekennzeichnet, dass** das Wasser vor der Herstellung des Kontakts mit der wasserfreien verbrauchbaren Vorrichtung erhitzt wird und/oder die Mischung während des Schritts des Mischens erhitzt wird, um die Bildung der Emulsion zu erleichtern, und/oder die erhaltene Emulsion vor dem Auftragen auf die Haut abgekühlt oder abkühlen gelassen wird.

9. Verfahren gemäß einem der Ansprüche 7 bis 8, **dadurch gekennzeichnet, dass** die erhaltene kosmetische Emulsion eine Creme oder eine Milch oder ein Gel ist.

## Claims

1. An anhydrous device being in the form of a closed shell containing a fatty phase, **characterized in that**:
- the shell and the fatty phase constituting the necessary ingredients and in an amount adequate for the extemporaneous preparation, solely by adding water, of a single-use cosmetic emulsion,
- the shell serves as gelling agent and/or texturing agent, and
- the emulsion is in the form of a cream, a lotion or a gel.

2. The device according to claim 1, **characterized in that** it is a capsule, a casing, or a sealed sachet.

3. The device according to one of the preceding claims, **characterized in that** the ingredients contained in the fatty phase are chosen from among the cosmetic active ingredients, synthetic oils, vegetable oils, emollients, anti-UV filters, texturing agents, thickening agents, gelling agents, pigments or dyes, aromas or fragrances, surfactants.

4. The device according to one of claims 1 to 3, **characterized in that** the shell is made from one or more components from among gelatin, hydroxy-propyl methyl cellulose, natural or synthetic polymers, pullulan or gellan gum.

5. The device according to one of the preceding claims, **characterized in that** the ingredients contained in the fatty phase are in a solid, liquid or pasty form.

6. A method for extemporaneous preparation of a single-use cosmetic emulsion implementing the device according to one of claims 1 to 5.

7. The method according to claim 6, **characterized in that** it comprises the following steps:
- providing the consumable anhydrous device comprising a fatty phase,
- providing water, in a predetermined amount, corresponding to the amount necessary for the preparation of the single-use cosmetic emulsion;
- putting water in contact with the device and mixing the whole to form a single-use cosmetic emulsion applicable directly, immediately and in its entirety on the skin, the skin appendages or the mucous membranes.

8. The method of claim 7, **characterized in that** water is heated before putting it in contact with the consumable anhydrous device and/or heating the mixture during the mixing step to facilitate the formation of the emulsion, and/or cooling or allowing the emulsion obtained to cool down before applying it on the skin.

9. The method according to one of claims 7 to 8, **characterized in that** the cosmetic emulsion obtained is either a cream or a lotion.
